# EUROPEAN PATENT SPECIFICATION

(11) **EP 1 949 895 B1**
(45) Date of publication and mention of the grant of the patent: **09.03.2016**
(21) Application number: 06823260.2
(22) Date of filing: 09.11.2006
(51) Int. Cl.: A61K 9/08, A61K 31/137, A61K 31/167, A61K 45/06, A61K 47/32, A61P 23/02

(54) **IONTOPHORESIS PREPARATION**
IONTOPHORESE-ZUBEREITUNG
PREPARATION POUR IONTOPHORESE

(30) Priority: 14.11.2005 US 735830 P
(43) Date of publication of application: 30.07.2008
(73) Proprietor: TEIKOKU SEIYAKU CO., LTD., Higashikagawa-shi, Kagawa 769-2695 (JP)
(72) Inventor: GOTO, Takeshi, Ibaraki, 3001206 (JP); MORI, Kenji, Ibaraki, 3010043 (JP)
(74) Representative: Beckmann, Claus
(86) International application number: PCT/JP2006/322373
(87) International publication number: WO 2007/055279

(56) References cited:
- EP-A1- 0 643 981
- EP-A1- 1 133 985
- EP-A1- 1 547 579
- WO-A1-98/35722
- WO-A1-2004/019902
- WO-A2-02/02182
- WO-A2-98/20869
- JP-A- 05 097 662
- JP-A- 07 213 627
- JP-A- 09 286 891
- JP-A- 11 076 428
- JP-A- 11 099 209
- JP-A- 2000 219 623
- JP-A- 2002 114 711
- JP-A- 2003 534 107
- JP-A- 2004 501 727

## Description

### FIELD OF INVENTION

The present invention relates to a preparation containing a drug used for an iontophoretic device which is used for administering a physiologically active substance via the skin or mucosa by means of an electric energy, and this system is mainly used in medical field.

### BACKGROUND ART

There have been many studies for absorbing a physiologically active substance such as a medicament via the skin or mucosa and many drugs prepared by utilizing these techniques have been commercialized.

However, as part of the skin and mucosa covers the most outer surface of a body and works as a barrier to protect invasion of foreign substances, the permeability of the substance is little. Therefore, it is difficult to deliver a physiologically active substance into a body in an amount enough to show the therapeutic effect by only applying the said substance to the skin or mucosa. As such, there are many studies on methods for enhancement of the absorption such as utilizing absorption enhancers or outer energy.

As a method for utilizing outer energy, there is reported iontophoresis which uses electric energy (Journal of Pharmaceutical Science, Vol. 6, p. 341, 1987, U.S. Patent No. 4,141,359). Iontophoresis is a system that generally a preparation containing an electrode and a drug such as a physiologically acceptable substance (a drug reservoir) and a counter electrode containing an electrode and ion such as sodium chloride are applied to the skin in a distance of several cm or several ten cm, and electric current from some microampere to some milliampere between both electrodes is applied for several minutes to several hours. By the electric current then occurred, a drug such as a physiologically active substance is delivered from the skin or mucosa into a body. According to this technique, not only an ionic lower molecule weight substance, but also a nonionic and aqueous substance such as mannitol or a high molecular weight substance such as calcitonin can be absorbed.

An iontophoretic device containing a local anesthetic, lidocaine in a drug reservoir has been sold in USA.

However, in such a preparation, usually a drug, etc. are dissolved or partially dissolved in a solvent containing mainly water and therefore, the stability of those substances are anxious. Therefore, developed is the preparation that the substances are kept in dry without dissolving in the solvent and the substances are dissolved when using (WO 98/13099). According to such a preparation, it is necessary to dissolve the substances in need and it is troublesome for a patient to utilize it. Depending on the substances in dry, it may be necessary to increase the rate of the solubility.

With regard to the commercialized device mentioned above, unstable epinephrine is contained therein in order to enhance the activity of lidocaine, and a stabilizer such as disulfite for unstable epinephrine is also contained to cause decrease of the absorption rate of a main drug, lidocaine.

WO 98/20869 A2 discloses a composition for iontophoresis comprising 1-10% lidocaine, 0.01-0.2% epinephrine and one or more antioxidants or metal chelators.

EP 1 133 985 A1 discloses in its Example 3 an adhesive gel composition for iontophoresis comprising lidocaine and epinephrine.

WO 02/02182 A2 discloses a reservoir-electrode for iontophoresis comprising lidocaine and epinephrine.

EP 1 547 579 A1 discloses an adhesive gel composition for iontophoresis comprising lidocaine (see Examples 29-31).

EP 0 643 981 A1 discloses lidocaine as a drug for use in a matrix for iontophoresis at page 8, lines 4-6. Further, this reference discloses chlorobutanol as an ingredient which may be contained in the matrix (see page 6, lines 16-20), however, chlorobutanol is described as a preservative and not as a stabilizer for epinephrine.

### DISCLOSURE OF INVENTION

As mentioned above, in the preparation containing such a drug as becoming unstable under iontophoresis, it was a problem to construct a preparation that even if water and an unstable substance co-exist, the stability is maintained and the absorption rate of a main drug is not reduced.

The present inventors, in order to obviate the problem, have selected epinephrine as an unstable substance in the presence of water and lidocaine as a main drug, and have studied additives to stabilize epinephrine and not to affect (decrease) the absorption of lidocaine under iontophoresis, and as a result, have found that by adding chlorobutanol thereto, epinephrines is unexpectedly stabilized, and the absorption rate of lidocaine is not reduced. Thus the present invention was completed.

In addition, when the preparation composition for iontophoresis is air-tightly sealed under an atmosphere of oxygen 5% or less, or is air-tightly sealed with a deoxidant, it was found that the effect on chlorobutanol is further strengthened.

"Air-tightly sealed" means that a content layer containing the preparation and its outside are completely separated by a sealing material such as aluminum sheet and there is no exchange of air between the content layer and its outside.

The present invention relates to a preparation composition containing a drug for an iontophoretic device, and said preparation composition stabilized by adding chlorobutanol to the preparation containing an unstable substance in the presence of water, and which does not reduce the absorption rate of a main drug under iontophoresis, and the preparation composition containing a substance (may be also a main drug) stabilized by chlorobutanol and a main drug for iontophoresis.

The present inventions are illustratively shown below.
(1) A preparation containing a drug used for iontophoresis for absorbing a physiologically active substance via the skin or mucosa by means of an electric driving force, and said preparation containing a local anesthetic, epinephrine or its salt, water and chlorobutanol.
(2) The preparation described in (1) wherein the local anesthetic is lidocaine or its salt.
(3) The preparation described in (1) or (2) wherein the content of chlorobutanol is 0.3 to 1.0w/w%.
(4) The preparation described in any one of (1) to (3) wherein at least one additive selected from disulfite (pyrosulfite), hydrogensulfite (bisulfite), edetic acid and edetate is further contained.
(5) The preparation described in (4) wherein the additive is sodium pyrosulfite.
(6) The preparation described in (4) wherein the additive is sodium bisulfite.
(7) The preparation described in any one of (1) to (6) wherein polyvinyl alcohol is further contained.
(8) The preparation described in any one of (1) to (7) wherein a deoxidant is together enclosed when the preparation is air-tightly sealed.
(9) The preparation described in any one of (1) to (7) wherein the preparation is air-tightly sealed under an atmosphere of 5% or less oxygen.
(10) A method of stabilizing a preparation for iontophoresis containing a local anesthetic, especially lidocaine, epinephrine or its salt, and water characterized in that chlorobutanol is added to said preparation.
(11) The method described in (10) wherein the preparation is air-tightly sealed.
(12) The method described in (10) wherein the preparation is air-tightly sealed under an atmosphere of 5% or less oxygen.
(13) The method described in (10) wherein at least one additive selected from disulfite, hydrogensulfite, edetic acid and edetate is further added to the preparation.
(14) The method described in (13) wherein the preparation is air-tightly sealed with a deoxidant.
(15) The method described in (13) wherein the preparation is air-tightly sealed under an atmosphere of 5% or less oxygen.

The present invention has an effect to stabilize a substance readily resolved in the presence of water without reducing the absorption rate of a main drug in a preparation used for iontophoresis.

### BRIEF DESCRIPTION OF THE DRAWINGS

Fig. 1: Cross section of a device for in vitro permeation study
Fig. 2: Relationship between content of an additive and permeation rate of lidocaine
Fig. 3: Comparison with skin permeation rate of lidocaine in case of a single additive and in case of combination of chlorobutanol and an additive
Fig. 4: Stability of epinephrine maintained at 60°C for 4 weeks
Fig. 5: Side view of the support of a preparation
Fig. 6: Upper view of the support of a preparation
Fig. 7: Side view of a preparation
Fig. 8: Relationship with content of chlorobutanol and stability of epinephrine
Fig. 9: Stabilization on epinephrine for long term
Fig. 10: Stabilization on epinephrine for long term

### Explanation of signals:

11 Stirring bar
12 Donor compartment (drug reservoir)
13 Silver electrode
14 Extracted skin from hairless mouse
15 Silver/silver chloride electrode
16 Receiver compartment
17 Water jacket
51 Cavity
52 Adhesive
53 Silver electrode
54 Adhesive foam
61 Cavity
62 Silver electrode
63 Adhesive foam
71 Polyvinyl alcohol containing chlorobutanol etc.
72 Adhesive foam
73 Silver electrode
74 Adhesive foam

### BEST MODE FOR CARRYING OUT THE INVENTION

Chlorobutanol is used as an antibacterial agent for a solution, for example, as an antibacterial agent for an injection containing epinephrine, but chlorobutanol has been neither used as a stabilizer nor reported to have been used in a preparation for iontophoresis.

In general, by adding an additive in a preparation for iontophoresis, the absorption rate of a main drug is reduced. In addition, in case of a usual additive, the absorption rate of a main drug is reduced in proportion to increase of the content of the additive. However, it was surprisingly found that in case of chlorobutanol, the addition of it does not only affect the absorption rate of a main drug, and the increase of the amount of it does not also affect the absorption rate.

As an iontophoretic device for which the preparation of the present invention is used a known conventional device is utilized.

As an electrode for an iontophoretic device is used a known electrode, and a polarized one such as a carbon electrode may be used, but preferably such a nonpolarized one as having a silver electrode on anode (positive electrode).

As a negative electrode may be used a polarized electrode such as a carbon electrode, but preferably a non polarized electrode such as silver/silver chloride electrode may be used.

A substance which is unstable in co-existence of water includes epinephrine or its salt. Epinephrine is usually contained in order to enhance the activity of a local anesthetic, but as it is unstable in the presence of water, such a preparation has often troublesome.

As a main drug, a local anesthetic is used lidocaine hydrochloride, dibucaine or tetracaine, especially preferably lidocaine hydrochloride.

The amount of chlorobutanol is not limited, but usually 0.1∼1.5%, preferably 0.3∼1.0w/w%.

When in the preparation of the present invention, chlorobutanol and other known additive such as disulfite, or hydrogensulfite, especially sodium hydrogensulfite or sodium pyrosulfite are contained together, the stabilization effect increases.

In case of combination, the absorption rate of a main drug, lidocaine is reduced comparing with in case of no additive, but in case of addition of chlorobutanol, the absorption rate of lidocaine is not reduced comparing with in case of addition of sodium hydrogensulfite or sodium pyrosulfite, alone. Therefore, the reduction of the absorption rate of lidocaine is due to other stabilizer such as sodium hydrogensulfite or sodium pyrosulfite, etc., not due to chlorobutanol. In addition in case of addition of edetic acid or its salt, the same can be said.

When such an additive is used together with chlorobutanol, the additive is desirable to reduce the amount of the additive as possible, because the additive affects the absorption rate of a main drug,. For example, in case of sodium hydrogensulfite, the amount is 1% or less, preferably 0.3% or less. In case of sodium pyrosulfite, the amount is 1% or less, preferably 0.75% or less, more preferably 0.3% or less.

The preparation used for iontophoresis of the present invention is characterized by containing chlorobutanol, but the preparation may also contain following additives in addition to above additives.

For example, as ingredients for a base, are used hydrophilic substances such as polyvinyl alcohol, polyvinyl pyrrolidone, carboxyvinyl polymer, agar, xanthan gum, locust bean gum, and so on, preferably to prepare a gel preparation of the present invention. Polyvinyl alcohol is preferably used therefore. Other additives which are usually used in the preparation may be added thereto, if necessary, such as pH controller (citric acid, its salt, phosphoric acid, its salt, tartaric acid, its salt, acetic acid, its salt, boric acid, its salt, carbonate), benzoate, parahydroxybenzoates, ethanol, urea, glycerin, lactose, ascorbic acid, its esters, cysteine, tocopherol, thioglycolates, and so on. However, such additives as prohibiting the activity of chlorobutanol should be naturally avoided.

The present invention is explained below by illustrating examples and experiments, but the present invention should not be limited only by them.

### Example

Preparations of the present invention having ingredients shown in the following table 1-1 (Examples 1 to 8) were prepared.

**Table 1-1**

| | Example 1 | Example 2 | Example 3 | Example 4 | Example 5 | Example 6 | Example 7 | Example 8 |
|---|---|---|---|---|---|---|---|---|
| Lidocaine hydrochloride | 4.62 | 4.62 | 4.62 | 4.62 | 4.62 | 4.62 | 4.62 | 4.62 |
| Epinephrine tartrate | 0.091 | 0.091 | 0.091 | 0.091 | 0.091 | 0.091 | 0.091 | 0.091 |
| Disodium edetate | | | | | 0.8 | | | |
| Sodium pyrosulfite | | | | | | 0.75 | | 0.3 |
| Sodium bisulfite | | | | | | | 0.3 | |
| Chlorobutanol | 0.5 | 1.0 | 1.5 | 3 | 0.5 | 0.5 | 0.5 | 0.5 |
| Water | Residual | Residual | Residual | Residual | Residual | Residual | Residual | Residual |
| Total | 100 | 100 | 100 | 100 | 100 | 100 | 100 | 100 |

| | | | | | | | | |
|---|---|---|---|---|---|---|---|---|
| (Unit: w/w%) | | | | | | | | |

Preparations of the present invention having ingredients shown in the following table 1-2 (Examples 9 to 15) were prepared.

**Table 1-2**

| | Example 9 | Example 10 | Example 11 | Example 12 | Example 13 | Example 14 | Example 15 |
|---|---|---|---|---|---|---|---|
| Lidocaine hydrochloride | 4.62 | 4.62 | 4.62 | 4.62 | 4.62 | 4.62 | 4.62 |
| Epinephrine tartrate | 0.091 | 0.091 | 0.091 | 0.091 | 0.091 | 0.091 | 0.091 |
| Disodium edetate | | | | | 0.8 | | |
| Sodium pyrosulfite | 0.3 | 0.3 | 0.3 | | 0.3 | | |
| Sodium bisulfite | | | | 0.3 | | | |
| Chlorobutanol | 0.5 | 0.5 | 0.5 | 0.5 | 0.5 | 0.2 | 0.3 |
| Polyvinyl alcohol | 18 | 12 | 15 | 12 | 12 | | |
| Water | Residual | Residual | Residual | Residual | Residual | Residual | Residual |
| Total | 100 | 100 | 100 | 100 | 100 | 100 | 100 |

| | | | | | | | |
|---|---|---|---|---|---|---|---|
| (Unit: w/w%) | | | | | | | |

Preparations of the present invention having ingredients shown in the following tables 1 -3 and 1-4 (Examples 16 to 24) were prepared.

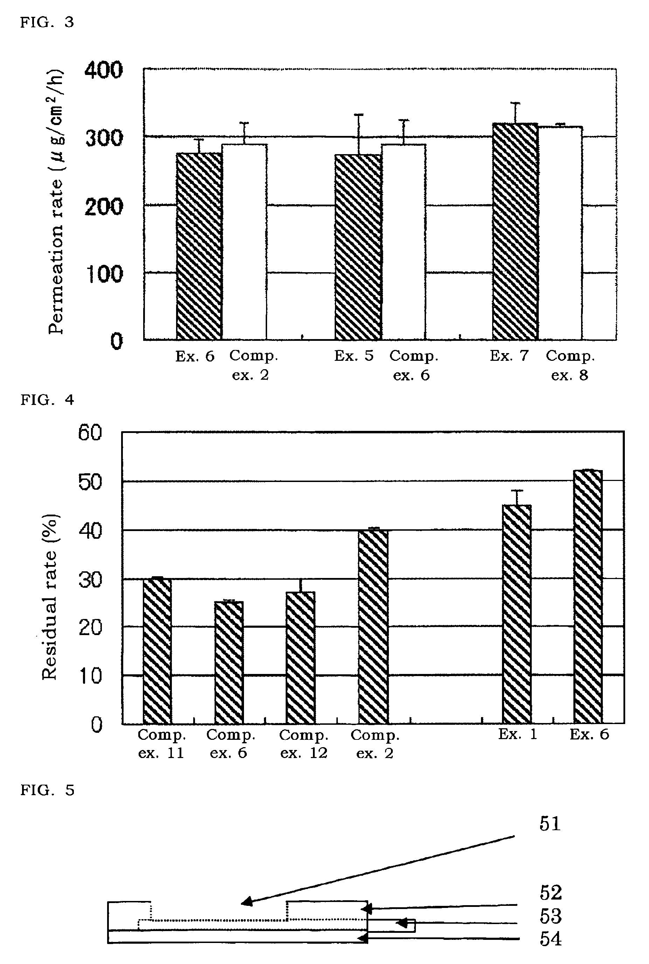

Namely, lidocaine hydrochloride, and polyvinyl alcohol and if necessary, glycerin, sodium pyrosulfite, or disodium edetate are mixed with a buffer in a sealed flask, and the mixture was heated for 20 minutes in a 80°C oil bath. After cooling to 30°C or less, thereto were added epinephrine tartrate dissolved in each buffer and chlorobutanol dissolved in ethanol, and the mixture was well mixed. A preparation thus prepared was weighting and about 1g of it was poured into a cavity shown in Fig. 5, followed by cooling at -80°C for about 12 hours. Then after the preparation was made gel by being allowed to stand at room temperature, liner was applied on the gel surface. The gel preparation was heat-sealed with a deoxidant (Ageless SA-200, Mitsubishi Gas Chemical) with a light shielded aluminum sheet.

**Table 1-3**

| | Example 16 | Example 17 | Example 18 | Example 19 | Example 20 |
|---|---|---|---|---|---|
| Lidocaine hydrochloride | 4.62 | 4.62 | 9.24 | 9.24 | 4.62 |
| Epinephrine tartrate | 0.091 | 0.091 | 0.182 | 0.182 | 0.091 |
| Disodium edetate | | | | | |
| Sodium pyrosulfite | 0.1 | | 0.1 | | 0.05 |
| Glycerin | | | | | |
| Polyvinyl alcohol | 15 | 15 | 15 | 15 | 15 |
| Chlorobutanol | 0.5 | 0.5 | 0.5 | 0.5 | 0.5 |
| Ethanol | 0.3 | 0.3 | 0.3 | 0.3 | 0.3 |
| 1/15mM citric acid buffer (pH2.6) | | Residual | | Residual | |
| 1/60mM phosphate buffer (pH2.0) | Residual | | Residual | | Residual |
| Total | 100 | 100 | 100 | 100 | 100 |

| | | | | | |
|---|---|---|---|---|---|
| (Unit: w/w%) | | | | | |

**Table 1-4**

| | Example 21 | Example 22 | Example 23 | Example 24 |
|---|---|---|---|---|
| Lidocaine hydrochloride | 4.62 | 4.62 | 9.24 | 9.24 |
| Epinephrine tartrate | 0.182 | 0.182 | 0.182 | 0.182 |
| Disodium edetate | | | 0.01 | 0.01 |
| Sodium pyrosulfite | 0.1 | | 0.1 | |
| Glycerin | | | 10 | 10 |
| Polyvinyl alcohol | 15 | 15 | 15 | 15 |
| Chlorobutanol | 0.5 | 0.5 | 0.5 | 0.5 |
| Ethanol | 0.3 | 0.3 | 0.3 | 0.3 |
| 1/15mM citrate buffer (pH2.6) | | Residual | | Residual |
| 1/60mM phosphate buffer (pH2.0) | Residual | | Residual | |
| Total | 100 | 100 | 100 | 100 |

| | | | | |
|---|---|---|---|---|
| (Unit: w/w%) | | | | |

Preparations of the present invention having ingredients shown in the following table 1-5 (Examples 25 and 26) were prepared in the similar manner as Examples 16 to 24. However, wrapping was carried out without any deoxidant. The preparation for Example 25 was carried out under an ordinal condition in a laboratory (at room temperature, oxygen content, 18%), and the preparation for Example 26 was carried out with heat-sealing under an atmosphere filled with nitrogen gas and oxygen content, 5% or less in a globe box.

**Table 1-5**

| | Example 25 | Example 26 |
|---|---|---|
| Lidocaine hydrochloride | 9.24 | 9.24 |
| Epinephrine tartrate | 0.182 | 0.182 |
| Disodium edetate | 0.01 | 0.01 |
| Glycerin | 10 | 10 |
| Polyvinyl alcohol | 15 | 15 |
| Chlorobutanol | 0.5 | 0.5 |
| Ethanol | 0.3 | 0.3 |
| 1/15mM citrate buffer (pH2.6) | Residual | Residual |
| Total | 100 | 100 |
| Wrapping method | Ordinal circumstance | Oxygen content 5% or less |

| | | |
|---|---|---|
| (Unit: w/w%) | | |

### Comparative example

Preparations having ingredients shown in the following table 2 (Comparative examples 1 to 11) were prepared and the preparations were subjected to the following experiments.

**Table 2**

| | Comp. ex. 1 | Comp. ex. 2 | Comp. ex. 3 | Comp. ex. 4 | Comp. ex. 5 | Comp. ex. 6 | Comp. ex. 7 | Comp. ex. 8 | Comp. ex. 9 | Comp. ex.10 | Comp. ex. 11 |
|---|---|---|---|---|---|---|---|---|---|---|---|
| Lidocaine hydrochloride | 4.62 | 4.62 | 4.62 | 4.62 | 4.62 | 4.62 | 4.62 | 4.62 | 4.62 | 4.62 | 4.62 |
| Epinephrine tartrate | 0.091 | 0.091 | 0.091 | 0.091 | 0.091 | 0.091 | 0.091 | 0.091 | 0.091 | 0.091 | 0.091 |
| Disodium edetate | | | | | 0.3 | 0.8 | 3.0 | | | | |
| Sodium pyrosulfite | 0.3 | 0.75 | 1.5 | 3.0 | | | | | | | |
| Sodium bisulfite | | | | | | | | 0.3 | 1.5 | 3.0 | |
| Chlorobutanol | | | | | | | | | | | |
| Water | Residual | Residual | Residual | Residual | Residual | Residual | Residual | Residual | Residual | Residual | Residual |
| Total | 100 | 100 | 100 | 100 | 100 | 100 | 100 | 100 | 100 | 100 | 100 |

| | | | | | | | | | | | |
|---|---|---|---|---|---|---|---|---|---|---|---|
| (Unit: w/w%) | | | | | | | | | | | |

### Comparative example 12

Lidocaine hydrochloride (4.62%), epinephrine tartrate (0.091%), n-propyl gallate (0.2%) and water (residue) were mixed to prepare a solution and then, was subjected to the comparative experience.

### Comparative example 13

A gel preparation (Example 16) containing lidocaine hydrochloride (4.62%), epinephrine (0.091%), polyvinyl alcohol (15%) and water (residue) were prepared in the same manner as Example 16, and be subjected to the comparative experiment below.

Usefulness on chlorobutanol in a preparation composition used for iontophoresis is explained by illustrating the stabilization effect against epinephrine and the effect on permeability of lidocaine.

### Experiment

The following experiment 1 was carried out on the effect on permeability of lidocaine.

### Experiment 1

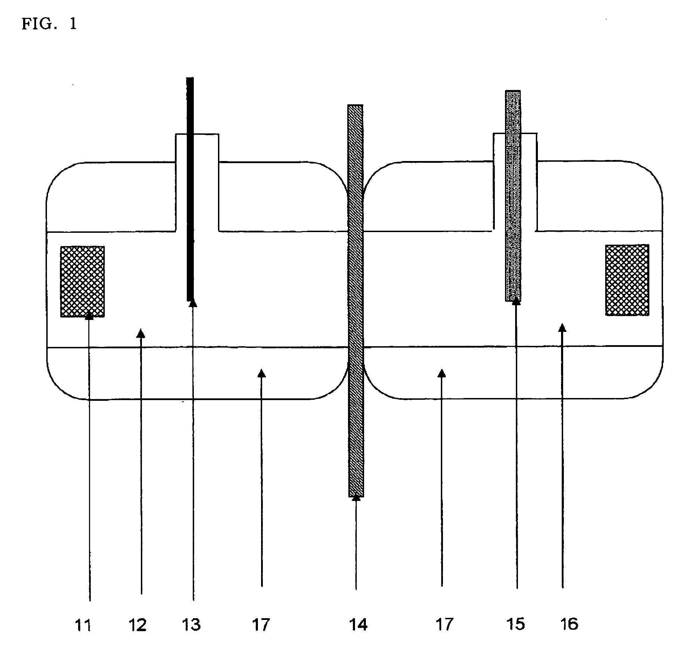

As shown in Fig. 1, a skin (14) extracted from a hairless mouse was set to an oblong 2-chamber cell for permeation test (effective permeation area 0.95cm²) warmed to 37°C by water jacket (17). Each test solution (Example 1 to 7 and comparative examples 1 to 11) (3ml) was applied to a donor site (12) and 0.9% aqueous sodium chloride solution (3ml) was applied to a receiver layer. Silver electrode (13) was connected to a donor site and silver/silver chloride electrode (15) prepared by electrolysis was connected to a receiver side, and constantly applied current in 0.5mA by a direct voltage current generation (Precise Gauge VI-1002). Every one hour, receiver solution (1ml) was collected and the content of lidocaine therein was measured and its permeation rate was calculated.

### Consideration

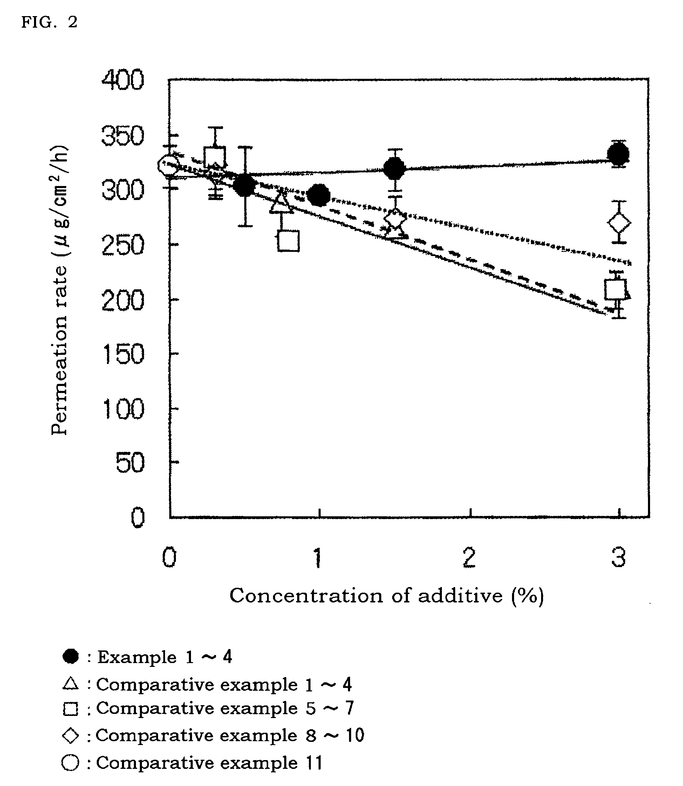

As shown in Fig. 2, in Examples 1 to 4, even in case of addition of chlorobutanol and even in case of change of the amount thereof, the permeation rate of lidocaine was not changed under iontophoresis, and the permeation rate showed about 300µg/cm²/h or more and its rate was almost constant (the rate was almost the same as in case of no additive (Comparative example 11). On the other hand, in the solutions on Comparative examples 1 to 10, the rate of permeability of lidocaine was reduced in proportion to increase of the amount of sodium pyrosulfite, disodium edetate or sodium bisulfite and when sodium pyrosulfite or disodium edetate was added in the amount of 3%, the rate of permeability was reduced to 200µg/cm²/h and in case of addition of sodium biulfite in the amount of 3%, the rate was reduced to 270µg/cm²/h.

Furthermore, the effect on the rate of the permeability of lidocaine by addition of chlorobutanol and other additive was shown in Fig. 3. The rate of permeability of lidocaine was almost the same as in case of only addition of other additive than chlorobutanol (Comparative examples 2, 6 and 8) as in Examples 5 to 7 even in case of addition of chlorobutanol. Namely chlorobutanol different from other additive does not effect the rate of permeability of the main drug and is suitable for iontophoresis.

The following experiments 2 and 3 were conducted on the effect on the stability on epinephrine.

### Experiment 2

The solutions (each 1ml) of Examples 1, 6 and Comparative examples 2, 6, 11, and 12 were poured into an ampoule and sealed. The preparation was maintained at such severe conditions as at 60°C, relative humidity 75% for 4 weeks and then the amount of epinephrine in the ampoule was measured, and its residual amount to its initial amount was calculated.

### Consideration

As shown in Fig. 4, in case of no additive as Comparative example 11, the residual amount of epinephrine was reduced by 30% from its initial amount. Even when edetate (Comparative example 6) or n-propyl gallate (Comparative example 12), which is generally classified to a stabilizer was added, the residual rate of epinephrine was almost the same as the rate in Comparative example 11 or smaller. In case of addition of sodium pyrosulfite (Comparative example 2) classified to a stabilizer as well, the residual rate was improved by about 10%. On the other hand, in the solution containing chlorobutanol (Example 1), the residual rate was to about 45% and the stability of epinephrine was improved. In case of combination of sodium pyrosulfite and chlorobutanol (Example 6), the residual rate showed more than 50%, which is higher than the rate in case of a sole additive. From this result, it was shown that addition of chlorobutanol causes to extremely increase the stability of epinephrine.

### Experiment 3

Polyvinyl alcohol-suspensions containing the ingredients shown in Examples 9 to 13 were prepared and after weighing the weight, the suspensions were heated at 120°C for 20 minutes to give viscous solutions. Thereto was added the same amount of water as distilled water, and the mixtures were cooled to room temperature to give gel preparations.

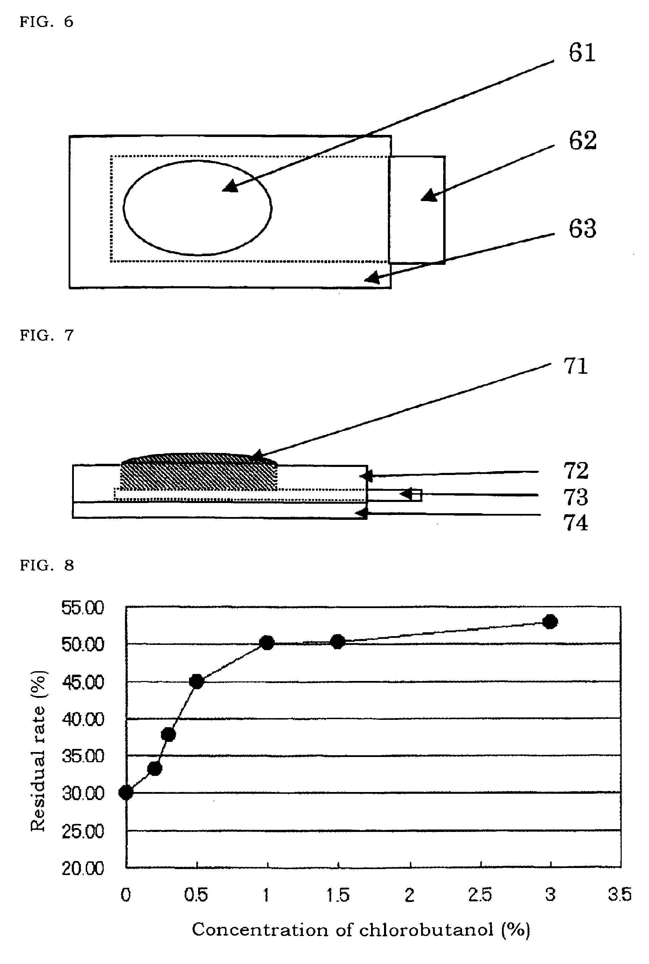

Each of the gels was added to a cavity of the preparation -support constructed with adhesive foam (52, 54, 63) and a silver electrode (53, 62) as shown in Fig. 5 (side view) and Fig. 6 (upper view) and was cooled to -80°C for 24 hours. And then taken out, it was allowed to stand for one hour and observed. Dotted lines in Fig. 6 and 7 mean hidden lines.

### Consideration

In any of Examples 9 to 13, there was obtained a preparation with suitable viscosity used for iontophoresis. A preparation used for iontophoresis as shown in Fig. 7 (Dotted line in Fig. means hidden line, and pale slant line part shows an inner gel part which can not be actually seen from outside when the preparation is completed.). Namely, a preparation containing chlorobutanol used for iontophoresis which can be utilized in practice could be prepared by using polyvinyl alcohol.

The following experiment 4 was carried out on a relation ship between the content of chlorobutanol and the stability of epinephrine.

### Experiment 4

In the same manner as Experiment 2, the residual rate of epinephrine was tested on Examples 2 to 4, 14 and 15.

### Consideration

The residual rate of epinephrine after maintaining at 60°C for 4 weeks was shown in Fig. 8. The effect of addition of chlorobutanol to the stabilization of epinephrine exerts at the content of 0.2% and at the content of 0.3% or more, the effect is evidently confirmed, and further by 1.0%, the stability of epinephrine was increased in proportion with increase of the content of chlorobutanol. Thereafter by 3% the effect was almost constant. Namely, the effect of addition of chlorobutanol was confirmed in any concentration, but in considering of relative effect of concentration of an additive, the content of 0.3%∼1.0% was found preferable.

The following experiment 5 was carried out the stability on epinephrine for a longer term.

### Experiment 5

Preparations (Examples 16 to 24) were maintained at 40°C, and relative humidity 75% for 6 months. Then the content of epinephrine in the preparations was measured to study its stability. Extracting method of epinephrine from the gel and measuring method of epinephrine were shown below.

### Extracting method

To the gel weighted were added 60mM phosphate buffer (pH2.0) and an inner standard solution (methylparaben: 150mg/ml), and the mixture was agitated at 80°C for 1 hour in an incubator to dissolve the gel. After being cooled, thereto was added methanol until its amount became more than 80%, and the mixture was agitated at room temperature for 30 minutes so as the precipitates do not aggregate. The definite amount was taken and measured and after filtration was subjected to a sample for HPLC.

### Quantitative method

The amount of epinephrine was measured by HPLC system (LC-10A: Shimadzu Corporation).
Column: Tosoh TSKgel ODS-100V (250mm long x 4.6mm i.d.)
Mobile phase solution: Phosphate buffer 35% (pH2.1) containing acetonitrile 65% and 10mM sodium dodecylsulfate
Flow rate: 1.5mL/min.
Detection: Ultraviolet detector (Shimadzu SPD-10A) The amount was measured at 281nm.
Preparations (Comparative example 13) was maintained at 40°C, and relative humidity 75% for 2 months. Then epinephrine was extracted and was measured to study its stability, in the same manner as Example 16.

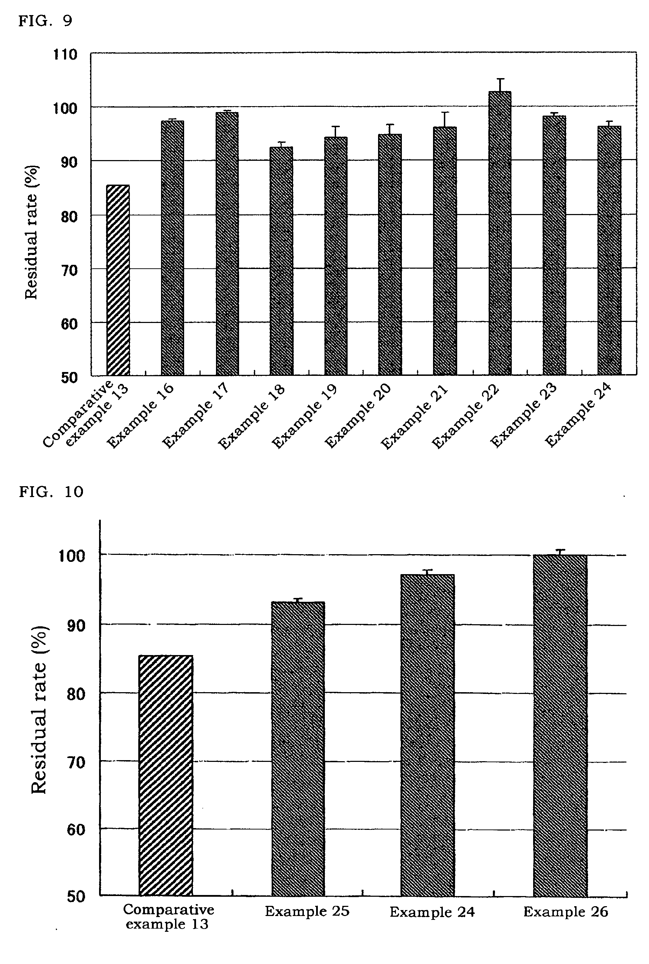

The result was shown in Fig. 9.

### Consideration

As shown in Fig. 9, the amount of epinephrine in the preparation of Comparative example 13 was reduced by 85.3% in 2 months, but the amount of epinephrine were maintained in more than 90% for 6 months in any preparations of Examples 16 to 24. Namely, preparations containing chlorobutanol (Examples 16 to 24) showed increase of the stability of epinephrine.

### Experiment 6

Preparations (Examples 24 to 26) were maintained at 40°C, and relative humidity 75% for 2 months. Then epinephrine was extracted and the residual amount of epinephrine was measured in the same manner as Experiment 5.

As shown in Fig. 10, in a preparation not containing chlorobutanol such as a preparation of Comparative example 13, the amount of epinephrine was only 85.3% in 2 months, but in the preparation containing chlorobutanol such as a preparation of Example 25 the residual amount of epinephrine was 93.1%, and increase of the stability was observed. Furthermore, when the preparation containing chlorobutanol was wrapped by heat sealing with a deoxidant such as Example 24, the amount of epinephrine was 97.1% and when a preparation containing chlorobutanol was wrapped under a condition of oxygen content less than 5% such as Example 26, the residual amount of epinephrine was to 100%. By combining addition of chlorobutanol and the packing method, it was found that the stability of the drug was unexpectedly increased.

## Claims

1. A preparation for iontophoresis containing a local anesthetic, epinephrine or its salt, water and chlorobutanol.

2. The preparation according to claim 1 wherein the local anesthetic is lidocaine or its salt.

3. The preparation according to claim 1 or 2 wherein the content of chlorobutanol is 0.3 to 1.0w/w%.

4. The preparation according to any one of claims 1 to 3 wherein at least one additive selected from disulfite, hydrogensulfite, edetic acid and edetate is further contained.

5. The preparation according to claim 4 wherein the additive is sodium pyrosulfite.

6. The preparation according to claim 4 wherein the additive is sodium bisulfite.

7. The preparation according to any one of claims 1 to 6 wherein polyvinyl alcohol is further contained.

8. The preparation according to any one of claims 1 to 7 wherein a deoxidant is together enclosed when the preparation is air-tightly sealed.

9. The preparation according to any one of claims 1 to 7 wherein the preparation is air-tightly sealed under an atmosphere of 5% or less oxygen.

10. A method of stabilizing a preparation for iontophoresis containing a local anesthetic, especially lidocaine, epinephrine or its salt, and water **characterized in that** chlorobutanol is added to said preparation.

11. The method according to claim 10 wherein the preparation is air-tightly sealed.

12. The method according to claim 10 wherein the preparation is air-tightly sealed under an atmosphere of 5% or less oxygen.

13. The method according to claim 10 wherein at least one additive selected from disulfite, hydrogensulfite, edetic acid and edetate is further added to the preparation.

14. The method according to claim 13 wherein the preparation is air-tightly sealed with a deoxidant.

15. The method according to claim 13 wherein the preparation is air-tightly sealed under an atmosphere of 5% or less oxygen.

## Patentansprüche

1. Zubereitung für die Iontophorese, enthaltend ein Lokalanästhetikum, Epinephrin oder dessen Salz, Wasser und Chlorbutanol.

2. Zubereitung nach Anspruch 1, wobei das Lokalanästhetikum Lidocain oder dessen Salz ist.

3. Zubereitung nach Anspruch 1 oder 2, wobei der Gehalt an Chlorbutanol 0,3 bis 1,0 G/G% ist.

4. Zubereitung nach einem der Ansprüche 1 bis 3, wobei des Weiteren mindestens ein Additiv, ausgewählt aus Disulfit, Hydrogensulfit, Edetinsäure und Edetat enthalten ist.

5. Zubereitung nach Anspruch 4, wobei das Additiv Natriumpyrosulfit ist.

6. Zubereitung nach Anspruch 4, wobei das Additiv Natriumbisulfit ist.

7. Zubereitung nach einem der Ansprüche 1 bis 6, wobei des Weiteren Polyvinylalkohol enthalten ist.

8. Zubereitung nach einem der Ansprüche 1 bis 7, wobei damit zusammen ein Deoxidans eingeschlossen ist wenn die Zubereitung luftdicht versiegelt ist.

9. Zubereitung nach einem der Ansprüche 1 bis 7, wobei die Zubereitung luftdicht versiegelt ist unter einer Atmosphäre von 5% oder weniger Sauerstoff.

10. Verfahren zur Stabilisierung einer Zubereitung für die Iontophorese, enthaltend ein Lokalanästhetikum, insbesondere Lidocain, Epinephrin oder dessen Salz und Wasser, **dadurch gekennzeichnet, dass** Chlorbutanol zu der Zubereitung zugegeben wird.

11. Verfahren nach Anspruch 10, wobei die Zubereitung luftdicht versiegelt wird.

12. Verfahren nach Anspruch 10, wobei die Zubereitung luftdicht versiegelt wird unter einer Atmosphäre von 5% oder weniger Sauerstoff.

13. Verfahren nach Anspruch 10, wobei des Weiteren mindestens ein Additiv, ausgewählt aus Disulfit, Hydrogensulfit, Edetinsäure und Edetat zu der Zubereitung zugegeben wird.

14. Verfahren nach Anspruch 13, wobei die Zubereitung luftdicht versiegelt wird mit einem Deoxidans.

15. Verfahren nach Anspruch 13, wobei die Zubereitung luftdicht versiegelt wird unter einer Atmosphäre von 5% oder weniger Sauerstoff.

## Revendications

1. Préparation pour iontophorèse contenant un anesthésique local, de l'épinéphrine ou son sel, de l'eau, et du chlorobutanol.

2. Préparation selon la revendication 1, dans laquelle l'anesthésique local est la lidocaine ou son sel.

3. Préparation selon la revendication 1 ou 2, dans laquelle la teneur en chlorobutanol est de 0,3 à 1,0 % en poids.

4. Préparation selon l'une quelconque des revendications 1 à 3, qui contient en outre au moins un additif choisi parmi un bisulfite, un hydrogénosulfite, l'acide édétique, et un édétate.

5. Préparation selon la revendication 4, dans laquelle l'additif est le pyrosulfite de sodium.

6. Préparation selon la revendication 4, dans laquelle l'additif est le bisulfite de sodium.

7. Préparation selon l'une quelconque des revendications 1 à 6, qui contient en outre du poly(alcool vinylique).

8. Préparation selon l'une quelconque des revendications 1 à 7, à laquelle un désoxydant est incorporé quand la préparation est rendue étanche à l'air.

9. Préparation selon l'une quelconque des revendications 1 à 7, laquelle préparation est rendue étanche à l'air dans une atmosphère contenant 5 % ou moins d'oxygène.

10. Procédé pour stabiliser une préparation pour iontophorèse contenant un anesthésique local, en particulier la lidocaïne, de l'épinéphrine ou son sel, et de l'eau, **caractérisé en ce que** du chlorobutanol est ajouté à ladite préparation.

11. Procédé selon la revendication 10, dans lequel la préparation est rendue étanche à l'air.

12. Procédé selon la revendication 10, dans lequel la préparation est rendue étanche à l'air dans une atmosphère contenant 5 % ou moins d'oxygène.

13. Procédé selon la revendication 10, dans lequel au moins un additif choisi parmi un bisulfite, un hydrogénosulfite, l'acide édétique et un édétate, est en outre ajouté à la préparation.

14. Procédé selon la revendication 13, dans lequel la préparation est rendue étanche à l'air avec un désoxydant.

15. Procédé selon la revendication 13, dans lequel la préparation est rendue étanche à l'air dans une atmosphère contenant 5 % ou moins d'oxygène.
